# FASCICULE DE BREVET EUROPEEN

(11) **EP 1 232 744 B1**
(45) Date de publication et mention de la délivrance du brevet: **25.10.2006**
(21) Numéro de dépôt: 02290378.5
(22) Date de dépôt: 15.02.2002
(51) Int. Cl.: A61K 8/67, A61Q 19/00

(54) **Composition comportant de la vitamine C preparée durant l'application**
Mittel enthaltend während der Anwendung vorbereitetes Vitamin C
Composition containing vitamin C prepared during application

(30) Priorité: 19.02.2001 FR 0102234
(43) Date de publication de la demande: 21.08.2002
(73) Titulaire: L'ORÉAL, 75008 Paris (FR)
(72) Inventeur: Dalko, Maria, 91190 Gif sur Yvette (FR); Pereira, Rui, 37520 La Riche (FR)
(74) Mandataire: Vaillant, Jeanne

(56) Documents cités:
- EP-A- 0 476 442
- EP-A- 0 667 145
- EP-A- 0 970 691
- DE-A- 3 502 141
- GB-A- 763 055
- US-A- 6 060 512

## Description

La présente invention concerne des compositions comportant de la vitamine C obtenue par voie enzymatique, l'utilisation de certaines enzymes dans la préparation de compositions, et notamment de compositions cosmétiques ainsi qu'un procédé cosmétique mettant en oeuvre l'application de telles compositions sur la peau.

La vitamine C ou acide ascorbique est un actif, notamment un actif cosmétique, très intéressant. Il est connu pour stimuler la croissance du tissu conjonctif, notamment celle du collagène, et pour renforcer les défenses du tissu cutané contre les agressions extérieures. Il l'est également pour enlever les taches et pigmentations de la peau et favoriser la cicatrisation de la peau lésée.

Un problème crucial de cette molécule est toutefois son instabilité qui la rend difficile à formuler dans des compositions usuelles. De plus, après application des compositions, la vitamine C se dégrade rapidement.

De ce fait, il existe un besoin pour des compositions, notamment des compositions cosmétiques ou dermatologiques, comportant de l'acide ascorbique et qui remédient à ce problème de stabilité de l'actif.

Elle concerne une composition d'application topique à préparation extemporanée comportant de l'acide ascorbique et un support acceptable, l'acide ascorbique étant obtenu par mise en contact d'au moins un précurseur d'acide ascorbique, à l'exception des esters de l'acide ascorbique, et d'au moins une enzyme capable de convertir ledit précurseur.

Selon l'invention, la vitamine C est générée directement pendant ou juste avant son application sur la peau, ou même après l'application, c'est-à-dire juste avant son absorption.

Ce processus résout les problèmes de formulation inhérents à la vitamine C rencontrés jusqu'ici.

De plus, selon certains modes de mise en oeuvre, comme la transformation des précurseurs par l'enzyme peut être progressive, les compositions et le procédé de l'invention permettent d'obtenir une libération constante et régulière à la surface de la peau ; il y a par conséquent une meilleure bio-disponibilité *in vivo* et une efficacité plus importante des compositions.

En outre, la présente invention concerne l'utilisation d'une ou plusieurs enzymes choisies parmi la L-galactono-1,4-lactone deshydrogénase, la L-galactose deshydrogénase, la L-sorbosone deshydrogénase, la L-gulono-1,4-lactone oxydase ou leurs mélanges ou d'un extrait en comportant pour la préparation d'acide ascorbique à usage topique.

De plus, l'invention concerne un traitement cosmétique par application sur la peau d'une composition décrite ci-dessus et ci-après. Elle concerne également un procédé de traitement cosmétique de la peau consistant à appliquer sur la peau de façon simultanée ou décalée dans le temps au moins une enzyme capable de convertir en acide ascorbique un précurseur de l'acide ascorbique, et au moins un précurseur de l'acide ascorbique, à l'exception de ses esters.

Par précurseur selon l'invention, on entend tout précurseur chimique ou biologique de l'acide ascorbique. Sont bien entendu considérés comme des précurseurs tout sucre ou substrat susceptible d'être transformé en vitamine C par au moins une étape enzymatique, par exemple une étape d'oxydation, à l'exception des esters de l'acide ascorbique.

De tels précurseurs sont notamment certains sucres, et parmi les sucres notamment la L-galactono-1,4-lactone, la L-gulono-1,4-lactone, la D-glucorono-1,4-lactone, l'acide D-glucuronique, la D-mannose, l'acide D-galacturonique, le D-glucose, le D-galactose, le L-galactose ou leurs mélanges. De préférence, on utilise la L-galactono-1,4-lactone.

Par enzyme selon l'invention, on entend toute enzyme ou mélange d'enzymes capable(s) de convertir en vitamine C au moins un précurseur de vitamine C, et plus particulièrement, les précurseurs ci-dessus, cette ou ces enzymes pouvant, plus largement, se présenter sous l'une ou plusieurs des formes décrites ci-après.

Parmi ces enzymes, on peut citer notamment la L-galactono-1,4-lactone deshydrogénase, la L-galactose deshydrogénase, la L-sorbosone deshydrogénase et la L-gulono-1,4-lactone oxydase. On peut également utiliser leurs mélanges. De préférence, on utilise la L-galactono-1,4-lactone deshydrogénase.

Dans les compositions de l'invention, les enzymes utilisées peuvent être sous forme d'extrait brut, de solution d'enzyme(s) purifiée(s), d'enzyme(s) immobilisée(s) sur matrice, notamment sur matrice sol-gel, sous forme solide ou liquide, éventuellement lyophilisée(s), incluse(s) dans un dispositif à libération contrôlée, encapsulée(s) ou incluse(s) dans des liposomes ou toute autre forme dans laquelle l'enzyme ou les enzymes, d'une part, et le substrat, de l'autre, sont séparés avant leur mise en contact, juste avant ou au moment de leur application.

Ainsi, l'enzyme peut provenir d'extrait de végétaux, d'animaux, d'insectes ou encore de microorganismes, particulièrement de cellules, différenciées ou indifférenciées, obtenues *in vivo* ou *in vitro,* avant ou après modification génétique.

La plupart des plantes sont considérées comme source d'enzyme(s) appropriée(s). A titre d'exemple, on peut citer *Arabidopsis thaliana*, *Rosa sp.* et *Pea seedlin*. Comme exemple de microorganisme capable d'exprimer des enzymes adéquates, on peut citer *Aerobacter sp..*

L'extrait comprenant l'enzyme peut être un extrait préparé à partir de tout matériel, particulièrement des cellules, ledit matériel ayant été obtenu par culture *in vitro.* La culture des cellules *in vitro* permet d'obtenir un matériel standardisé et disponible tout au long de l'année contrairement à celui obtenu par culture *in vivo.*

Par culture *in* vitro, on entend l'ensemble des techniques connues de l'homme du métier qui permet de manière artificielle l'obtention d'un organisme (organe).

Dans le domaine du végétal, la culture *in vitro* apporte entre autres avantages celui que les végétaux ne sont plus soumis aux saisons mais sont accessibles toute l'année, répondant ainsi aux critères quantitatifs de l'industrie, et dans des conditions parfaitement reproductibles puisque la culture a lieu en conditions parfaitement contrôlées de température, de pH, de milieu de culture et répond au critère industriel de qualité.

Ainsi, par exemple, selon l'invention, l'extrait peut être un extrait d'organe comme les racines, tiges, feuilles, fleurs, pétales, fruits, voire de cellules d'organe, d'au moins un végétal obtenu par culture *in vitro* ou encore un extrait de cellules dédifférenciées.

Préférentiellement selon l'invention, on utilise des cellules végétales dédifférenciées.

Par cellules végétales dédifférenciées, on entend toute cellule végétale ne présentant aucun des caractères d'une spécialisation particulière et capable de vivre par elle-même et non en dépendance avec d'autres cellules. Ces cellules végétales dédifférenciées sont éventuellement aptes, sous l'effet d'une induction, à toute différenciation conforme à leur génome.

Selon la méthode de culture choisie, et en particulier selon le milieu de culture choisi, il est possible d'obtenir à partir d'un même explant des cellules végétales dédifférenciées présentant des caractères différents (Plant propagation by tissue culture, George E. F. and Sherrigton P. D., 1984, Exegetics Limited).

Par ailleurs, comme source d'enzymes clonées, on peut signaler la source décrite dans la demande de brevet WO 98/02 830.

Dans les compositions selon l'invention, la préparation de l'acide ascorbique est réalisée par mise en contact d'au moins une enzyme ou d'un extrait comportant au moins une enzyme avec au moins un substrat. Selon une première variante, on introduit l'enzyme ou les enzymes, d'une part, et le ou les précurseur(s), d'autre part, dans une composition unique, qui est de préférence préparée juste avant l'emploi.

Selon une seconde variante, l'enzyme ou les enzymes, d'une part, et le ou les précurseur(s), d'autre part, sont conditionnés de sorte qu'ils ne sont pas en contact, l'un avec l'autre, notamment avant l'emploi.

Par exemple, ils peuvent être incorporés dans deux compositions différentes destinées à être mélangées au moment de l'application ou appliquées de façon successive ou décalée dans le temps.

Ainsi, on peut disposer ces compositions dans deux compartiments séparés ; ils entrent néanmoins en communication par l'intermédiaire d'un conduit commun ; les compositions s'y mélangent et/ou peuvent sortir en se mélangeant, avant ou lors de l'application sur la peau.

De tels dispositifs de conditionnements en deux compartiments sont par exemple décrits dans les documents FR-A-2045559, FR-A-2105332, FR-A-2258319, FR-A-2293375, FR-A-2586913 ou FR-A-2643615.

Par ailleurs, on peut aussi réaliser au moins l'une des compositions sous forme encapsulée et/ou sous forme de microcapsules ou de microgranulés qui peuvent être incorporé(e)s dans un véhicule acceptable, par exemple dans l'autre composition.

Ainsi, l'enzyme et/ou le précurseur peuvent être sous forme encapsulée ou incluse dans des liposomes, sous forme de microcapsules ou de microgranulés.

Les microcapsules ou les microgranulés ou les liposomes sont écrasé(e)s au moment de l'application, ou au moment de la sortie de la composition de son emballage, par cisaillement ou par frottement sur la peau, par exemple, ce qui permet le mélange du ou des enzyme(s) et du ou des précurseur(s) au moment de l'application, et la production de la vitamine C directement sur la peau.

Par support ou milieu approprié pour une application topique, on entend tout milieu cosmétiquement et/ou dermatologiquement acceptable, c'est-à-dire compatible avec la peau et/ou les cheveux. Ces milieux comprennent usuellement de l'eau, ou un mélange d'eau et de corps gras, ou un mélange de corps gras.

Comme corps gras utilisables dans l'invention, on peut citer les huiles minérales (vaseline, huile minérale), les huiles végétales et leurs dérivés hydrogénés, les huiles animales, les huiles de synthèse, les huiles siliconées (diméthicone, cyclométhicone) et les huiles fluorées. Comme autres corps gras, on peut encore citer les alcools gras, les acides gras et les cires.

En particulier, les compositions peuvent se présenter sous forme de solutions aqueuses, alcooliques ou hydroalcooliques, de gels hydrophiles ou lipophiles, de microémulsions, d'émulsions eau-dans-huile ou huile-dans-eau ou eau-dans-huile-dans-eau ou huile-dans-eau-dans-huile ayant l'aspect d'une crème ou d'un gel, éventuellement aptes à mousser, sous forme d'aérosol, ou encore sous forme de dispersions vésiculaires contenant des lipides ioniques et/ou non ioniques. Ces formes galéniques sont préparées selon les méthodes usuelles des domaines considérés.

De façon connue, le milieu approprié pour une application topique selon l'invention peut contenir également des adjuvants habituels dans le domaine cosmétique ou dermatologique, tels que les gélifiants hydrophiles ou lipophiles, les tensioactifs, les actifs hydrophiles ou lipophiles, les conservateurs, les antioxydants, les solvants, les parfums, les charges, les filtres et les matières colorantes.

Les quantités des différents constituants des compositions selon l'invention sont celles classiquement utilisées dans les domaines considérés.

Dans les compositions de l'invention, l'enzyme est présente en une quantité allant de 0,05 à 30 % en poids, de préférence 0,1 à 10 % en poids, par rapport au poids total de la composition. Le précurseur est présent en une quantité allant de 0,01 à 50 % en poids, de préférence 0,1 à 10 % en poids par rapport au poids total de la composition.

La composition selon l'invention peut constituer notamment des produits de protection, de traitement ou de soin pour le visage, pour le cou, pour les mains ou pour le corps, des produits de bronzage artificiel ou des produits pour les cheveux, et notamment pour le soin du cuir chevelu, par exemple sous forme de lotions traitantes, de crèmes ou de gels.

Les exemples qui suivent sont donnés à titre illustratif, afin de mieux faire comprendre l'invention qui ne s'y limite donc pas. Les quantités indiquées sont des pourcentages en poids.

| **Exemple 1 : Crème de soin** | |
|---|---|
| *Phase huileuse :* | |
| Triceteareth-4 phosphate/sodium C₁₄-C₁₇ alkyl sec sulfonate (Hostacerin CG vendu par la société Hoescht Celanese) (tensioactif) | 6 % |
| Vaseline | 2 % |
| Huile minérale | 4 % |
| Diméthicone | 3 % |
| Cyclométhicone | 3 % |
| Diméthicone copolyol (tensioactif) | 1 % |
| Triclosan (conservateur) | 0,1 % |
| L-galactono-1,4-lactone | 1 % |

| *Phase aqueuse* | |
|---|---|
| Propylène glycol (hydratant) | 2 % |
| PEG-20 (organoleptique) | 1 % |
| L-galactono-1,4-lactone déshydrogénase | 1 % |
| Phénoxyéthanol (conservateur) | 0,4 % |
| Eau | qsp 100 % |

La L-galactono-1,4-lactone déshydrogénase est introduite dans la phase aqueuse sous forme encapsulée dans des microcapsules contenant aussi de l'atélocollagène et des glycosaminoglycannes.

Ces microcapsules sont immergées dans le reste des constituants après préparation de l'émulsion.

| **Exemple 2 : Crème** | |
|---|---|
| *Phase huileuse* | |
| Triceteareth-4 phosphate/sodium C₁₄-C₁₇ alkyl sec sulfonate (Hostacerin CG vendu par la société Hoescht Celanese) (tensioactif) | 6 % |
| Vaseline | 2 % |
| Huile minérale | 4 % |
| Diméthicone | 3 % |
| Cyclométhicone | 3 % |
| Diméthicone copolyol (tensioactif) | 1 % |
| Triclosan (conservateur) | 0,1 % |
| L-gulono-1,4-lactone | 2 % |

| *Phase aqueuse* | |
|---|---|
| Propylène glycol (hydratant) | 2 % |
| PEG-20 (organoleptique) | 1 % |
| L-gulono-1,4-lactone déshydrogénase | 2 % |
| Phénoxyéthanol (conservateur) | 0,4 % |
| Eau | qsp 100 % |

La L-gulono-1,4-lactone est introduite dans la composition sous forme de microsphères contenant aussi de l'atélocollagène et des glycosaminoglycannes.

Ces microsphères sont immergées dans le reste des constituants après préparation de l'émulsion.

| **Exemple 3 : Emulsion traitante** | |
|---|---|
| A. Emulsion contenant le précurseur : | |
| *Phase huileuse :* | |
| Steareth-2 (tensioactif) | 3 % |
| Steareth-21 (tensioactif) | 2 % |
| PPG-15 stearyl éther (tensioactif) | 29,5 % |
| L-galactono-1,4-lactone | 0,5 % |
| *Phase aqueuse :* | |
| Phénoxyéthanol (conservateur) | 0,5 % |
| Eau | qsp 100 % |
| | |

| B. Emulsion contenant l'enzyme : | |
|---|---|
| *Phase huileuse :* | |
| Steareth-2 (tensioactif) | 3 % |
| Steareth-21 (tensioactif) | 2 % |
| PPG-15 stearyl éther (tensioactif) | 29,5 % |
| *Phase aqueuse :* | |
| Phénoxyéthanol (conservateur) | 0,5 % |
| L-galactono-1,4-lactone déshydrogénase | 1 % |
| Eau | qsp 100 % |

Les émulsions A et B sont disposées dans deux compartiments séparés et mélangées au moment de l'application sur la peau.

| **Exemple 4 : Crème solaire autobronzante** | |
|---|---|
| A. Emulsion contenant l'ester de dihydroxyacétone : | |
| *Phase huileuse :* | |
| Steareth-2 (tensioactif) | 3 % |
| Steareth-21 (tensioactif) | 2 % |
| PPG-15 stearyl éther (tensioactif) | 29,5 % |
| L-galactose | 10 % |
| *Phase aqueuse :* | |
| Phénoxyéthanol (conservateur) | 0,5 % |
| Eau | qsp 100 % |

| B. Emulsion contenant la lipase : | |
|---|---|
| *Phase huileuse :* | |
| Steareth-2 (tensioactif) | 3 % |
| Steareth-21 (tensioactif) | 2 % |
| PPG-15 stearyl éther (tensioactif) | 29,5 % |
| *Phase aqueuse :* | |
| Phénoxyéthanol (conservateur) | 0,5 % |
| L-galactose déshydrogénase | 10 % |
| Eau | qsp 100 % |

Les émulsions sont disposées dans deux compartiments différents et sont mises en contact au moment de l'application.

| **Exemple 5 : Crème de soin** | |
|---|---|
| *Phase huileuse* | |
| Triceteareth-4 phosphate/sodium C₁₄-C₁₇ alkyl sec sulfonate (Hostacerin CG vendu par la société Hoescht Celanese) (tensioactif) | 5 % |
| Alcool stéarylique | 1 % |
| Vaseline | 2 % |
| Huile minérale | 4 % |
| Phényl triméthicone | 4 % |
| Cyclométhicone | 4 % |
| Diméthicone / Diméthiconol (tensioactif) | 2 % |
| Triclosan (conservateur) | 0,1 % |
| L-galactono-1,4-lactone | 0,6 % |

| *Phase aqueuse* | |
|---|---|
| Propylène glycol (hydratant) | 2 % |
| PEG-20 (organoleptique) | 1 % |
| L-galactono-1,4-lactone déshydrogénase | 0,5 % |
| Phénoxyéthanol (conservateur) | 0,2 % |
| Chlorphenesin | 0,2 % |
| Polyacrylamide/C13-C14 Isoparaffine/Laureth-7 (Sepigel 305 | |
| vendu par la société Seppic) (gélifiant) | 0,6 % |
| Eau | qsp 100 % |

La L-galactono-1,4-lactone est introduite dans la composition sous forme de microsphères contenant aussi de l'atélocollagène et du sodium chondroïtine sulfate.

Ces microsphères sont immergées dans le reste des constituants après préparation de l'émulsion.

| **Exemple 6 : Crème de soin pour la dépigmentation de la peau** | |
|---|---|
| A. Emulsion contenant le précurseur : | |
| *Phase huileuse :* | |
| Steareth-2 (tensioactif) | 3 % |
| Steareth-21 (tensioactif) | 2 % |
| PPG-15 stearyl éther (tensioactif) | 9 % |
| Alcool cétylique | 2 % |
| Vaseline | 5 % |
| Triclosan (conservateur) | 0,2 % |
| L-galactono-1,4-lactone | 1 % |

| *Phase aqueuse :* | |
|---|---|
| Propylène glycol (hydratant) | 4 % |
| PEG-20 (organoleptique) | 5 % |
| Phénoxyéthanol (conservateur) | 0,5 % |
| Eau | qsp 100 % |

| B. Emulsion contenant l'enzyme : | |
|---|---|
| *Phase huileuse :* | |
| Steareth-2 (tensioactif) | 3 % |
| Steareth-21 (tensioactif) | 2 % |
| PPG-15 stearyl éther (tensioactif) | 9 % |
| Alcool cétylique | 2 % |
| Vaseline | 5 % |
| Triclosan (conservateur) | 0,2 % |

| *Phase aqueuse :* | |
|---|---|
| Propylène glycol (hydratant) | 4 % |
| PEG-20 (organoleptique) | 5 % |
| Phénoxyéthanol (conservateur) | 0,5 % |
| L-galactono-1,4-lactone déshydrogénase | 1 % |
| Eau | qsp 100 % |

Les émulsions A et B sont disposées dans deux compartiments séparés et mises en contact au moment de l'application sur la peau.

## Revendications

1. Composition d'application topique pour la préparation extemporanée d'acide ascorbique, l'acide ascorbique étant obtenu par mise en contact d'au moins un précurseur d'acide ascorbique, et d'au moins une enzyme capable de convertir ledit précurseur, comportant, dans un support acceptable, au moins une enzyme ou un extrait comportant au moins une enzyme, l'enzyme étant choisie parmi la L-galactono-1,4-lactone deshydrogénase, la L-galactose deshydrogénase, la L-sorbosone deshydrogénase, la L-gulono-1,4-lactone oxydase ou leurs mélanges et un précurseur ou substrat choisi parmi les précurseurs chimiques ou biologiques de l'acide ascorbique, à l'exception de ses esters, l'enzyme et le précurseur étant conditionnés de sorte à ne pas être en contact l'un avec l'autre.

2. Composition selon la revendication 1, **caractérisée en ce que** le substrat est choisi parmi la L-galactono-1,4-lactone, la L-gulono-1,4-lactone, la D-glucorono-1,4-lactone, l'acide D-glucuronique, le D-mannose, l'acide D-galacturonique, le D-glucose, le D-galactose, le L-galactose, ou leurs mélanges.

3. Composition selon la revendication 1 ou 2, **caractérisée en ce que** le substrat est le L-galactono-1,4-lactone.

4. Composition selon l'une des revendications 1 à 3, **caractérisée en ce que** l'enzyme est la L-galactono-1,4-lactone deshydrogénase.

5. Composition selon l'une des revendications 1 à 4, **caractérisée en ce** l'enzyme provient d'extrait de végétaux, d'animaux, d'insectes ou encore de microorganismes, particulièrement de cellules, différenciées ou indifférenciées, obtenues *in vivo* ou *in vitro.*

6. Composition selon l'une des revendications 1 à 5, **caractérisée en ce que** l'enzyme et le précurseur sont conditionnés dans des compartiments séparés.

7. Composition selon l'une des revendications 1 à 6, **caractérisée en ce que** l'enzyme et/ou le précurseur sont sous forme encapsulée.

8. Composition selon l'une des revendications 1 à 7, **caractérisée en ce que** l'enzyme et/ou le précurseur sont sous forme de microcapsules ou de microgranulés.

9. Composition selon l'une des revendications 1 à 8, **caractérisée en ce que** l'enzyme est sous forme d'extrait total, de solution d'enzyme purifiée, d'enzyme immobilisée sur matrice, notamment sur matrice sol-gel, sous forme solide ou liquide, sous forme liquide ou solide lyophilisée ou incluse dans un dispositif à libération contrôlée.

10. Composition selon l'une des revendications 1 à 9, **caractérisée en ce que** l'enzyme est présente en une quantité allant de 0,05 à 30% en poids par rapport au poids total de la composition.

11. Composition selon l'une des revendications 1 à 10, **caractérisée en ce que** l'enzyme est présente en une quantité allant de 0,1 à 10% en poids par rapport au poids total de la composition.

12. Composition selon l'une des revendications 1 à 11, **caractérisée en ce que** le précurseur est présent en une quantité allant de 0,01 à 50% en poids par rapport au poids total de la composition.

13. Composition selon l'une des revendications 1 à 12, **caractérisée en ce que** le précurseur est présent en une quantité allant de 0,1 à 10% en poids par rapport au poids total de la composition.

14. Utilisation d'une ou plusieurs enzymes choisies parmi la L-galactono-1,4-lactone deshydrogénase, la L-galactose deshydrogénase, la L-sorbosone deshydrogénase, la L-gulono-1,4-lactone oxydase ou leurs mélanges ou d'un extrait en comportant pour la préparation d'acide ascorbique à usage topique, à partir d'un précurseur chimique ou biologique de l'acide ascorbique à l'exception de ses esters, au sein d'une composition d'application topique, l'enzyme et le précurseur étant conditionnés de sorte à ne pas être au contact l'un de l'autre.

15. Utilisation selon la revendication 14, **caractérisé en ce que** l'enzyme est la L-galactono-1,4-lactone deshydrogénase.

16. Utilisation selon la revendication 14 ou 15, **caractérisée en ce que** la préparation est extemporanée.

17. Utilisation selon l'une des revendications 14 à 16, **caractérisée en ce que** la préparation est réalisée par mise en contact de l'enzyme ou de l'extrait végétal avec un substrat.

18. Utilisation selon l'une des revendications 14 à 17, **caractérisée en ce** l'enzyme provient d'extrait de végétaux, d'animaux, d'insectes ou encore de microorganismes, particulièrement de cellules, différenciées ou indifférenciées, obtenues *in vivo* ou *in vitro.*

19. Utilisation selon l'une des revendications 14 à 18, **caractérisée en ce que** le substrat est choisi parmi la L-galactono-1,4-lactone, la L-gulono-1,4-lactone, la D-glucorono-1,4-lactone, l'acide D-glucuronique, le D-mannose, l'acide D-galacturonique, le D-glucose, le D-galactose, le L-galactose ou leurs mélanges.

20. Utilisation selon l'une des-revendications 14 à 19, **caractérisée en ce que** le substrat est la L-galactono-1,4-lactone.

21. Procédé de traitement cosmétique par application sur la peau d'une composition selon l'une des revendications 1 à 13.

22. Procédé de traitement cosmétique de la peau consistant à appliquer sur la peau de façon simultanée ou décalée dans le temps au moins une enzyme capable de convertir en acide ascorbique un précurseur de l'acide ascorbique, l'enzyme étant choisie parmi la L-galactono-1,4-lactone deshydrogénase, la L-galactose deshydrogénase, la L-sorbosone deshydrogénase, la L-gulono-1,4-lactone oxydase ou leurs mélanges et au moins un précurseur de l'acide ascorbique, à l'exception de ses esters, l'enzyme et le précurseur étant conditionnés de façon qu'ils ne soient pas en contact l'un avec l'autre.

23. Procédé selon la revendication 22, ce que l'enzyme est la L-galactono-1,4-lactone deshydrogénase.

24. Procédé selon la revendication 22 ou 23, **caractérisé en ce que** le précurseur est choisi parmi la L-galactono-1,4-lactone, la L-gulono-1,4-lactone, la D-glucorono-1,4-lactone, l'acide D-glucuronique, le D-mannose, l'acide D-galacturonique, le D-glucose, le D-galactose, le L-galactose ou leurs mélanges.

25. Procédé selon l'une des revendications 22 à 24, ce que le précurseur est la L-galactono-1,4-lactone.

## Claims

1. A topical composition application for extemporaneous preparation of ascorbic acid wherein the ascorbic acid is obtained by bringing at least one ascorbic acid precursor into contact with at least one enzyme that is capable of converting said precursor, said composition comprising in a suitable support, at least one enzyme or an extract comprising at least one enzyme, the enzyme being selected from L-galactono-1,4-lactone dehydrogenase, L-galactose dehydrogenase, L-sorbosone dehydrogenase, L-gulono-1,4-lactone oxidase and mixtures thereof, and a precursor or substrate selected from chemical or biological precursors of ascorbic acid, with the exception of its esters, wherein the enzyme and the precursor are packaged in such way as not to be in contact with each other.

2. The composition of claim 1, wherein said substrate is selected from L-galactono-1,4-lactone, L-gulono-1,4-lactone, D-glucorono-1,4-lactone, D-glucuronic acid, D-mannose, D-galacturonic acid, D-glucose, D-galactose, L-galactose or mixtures thereof.

3. The composition of claim 1 or 2, wherein the substrate is L-galactono-1,4-lactone.

4. The composition of one of claims 1 to 3, wherein the enzyme is L-galactono-1,4-lactone dehydrogenase.

5. The composition of one of claims 1 to 4, wherein the enzyme originates from an extract from plants, animals, insects or from micro-organisms, particularly differentiated or dedifferentiated cells obtained *in vivo* or *in vitro.*

6. The composition of one of claims 1 to 5, wherein the enzyme and the precursor are packaged in separate compartments.

7. The composition of one of claims 1 to 6, wherein the enzyme and/or the precursor are in an encapsulated form.

8. The composition of one of claims 1 to 7, wherein the enzyme and/or the precursor are in the form of microcapsules or microgranules.

9. The composition one of claims 1 to 8, wherein the enzyme is in the form of a total extract, a purified enzyme solution, an enzyme immobilised on a matrix, in particular on a sol-gel matrix, in the solid or liquid form, in the liquid or solid freeze-dried form, or included in a controlled release device.

10. The composition one of claims 1 to 9, wherein the enzyme is present in a quantity of 0.05% to 30% by weight with respect to the total composition weight.

11. The composition of one of claims 1 to 10, wherein the enzyme is present in a quantity of 0.1 % to 10% by weight with respect to the total composition weight.

12. The composition of one of claims 1 to 11, wherein said precursor is present in a quantity of 0.01% to 50% by weight with respect to the total composition weight.

13. The composition of one of claims 1 to 12, wherein the precursor is present in a quantity of 0.1 % to 10% by weight with respect to the total composition weight.

14. Use of one or more enzymes selected from L-galactono-1,4-lactone dehydrogenase, L-galactose dehydrogenase, L-sorbosone dehydrogenase, L-gulono-1,4-lactone oxidase and mixtures thereof or of an extract comprising said enzyme for the preparation of ascorbic acid for topical use, from a biological or chemical precursor of ascorbic acid, with the exception of its esters, within a composition for topical use, wherein the enzyme and the precursor are packaged in such a way as not to be in contact with each other.

15. The use of claim 14, wherein the enzyme is L-galactono-1,4-lactone dehydrogenase.

16. The use of claim 14 or 15 wherein the preparation is extemporaneous.

17. The use of claim 14 to 16, wherein the preparation is carried out by bringing the enzyme or plant extract into contact with a substrate.

18. The use of one of claims 14 to 17, wherein said enzyme originates from an extract from plants, animals, insects or from micro-organisms, particularly differentiated or undifferentiated cells obtained *in vivo* or *in vitro.*

19. The use of one of claims 14 to 18 wherein the substrate is selected from L-galactono-1,4-lactone, L-gulono-1,4-lactone, D-glucorono-1,4-lactone, D-glucuronic acid, D-mannose, D-galacturonic acid, D-glucose, D-galactose, L-galactose or mixtures thereof.

20. The use of one of claims 14 to 19, wherein said substrate is L-galactono-1,4-lactone.

21. A method for cosmetic treatment by the application to the skin of a composition according to one of claims 1 to 13.

22. A method for cosmetic treatment of the skin by the application to the skin, either simultaneously or successively, of at least one enzyme that can convert an ascorbic acid precursor into ascorbic acid, the enzyme being selected from L-galactono-1,4-lactone dehydrogenase, L-galactose dehydrogenase, L-sorbosone dehydrogenase, L-gulono-1,4-lactone oxidase and mixtures thereof, and at least one ascorbic acid precursor, with the exception of its esters, the enzyme and the precursor being packaged in such a way as not to be in contact with each other.

23. The method of claim 22, wherein said enzyme is L-galactono-1,4-lactone dehydrogenase.

24. The method of claim 22 or 23, wherein said precursor is selected from L-galactono-1,4-lactone, L-gulono-1,4-lactone, D-glucorono-1,4-lactone, D-glucuronic acid, D-mannose, D-galacturonic acid, D-glucose, D-galactose, L-galactose and mixtures thereof.

25. The method of one of claims 22 to 24, wherein said precursor is L-galactono-1,4-lactone.

## Patentansprüche

1. Zusammensetzung zur topischen Anwendung zur extemporalen Herstellung von Vitamin C, wobei das Vitamin C durch in Kontakt bringen mindestens eines Vitamin C-Vorläufers und mindestens eines Enzyms, das geeignet ist, den Vorläufer umzuwandeln, umfassend, in einem verträglichen Träger, mindestens ein Enzym oder einen Extrakt, der mindestens ein Enzym umfasst, wobei das Enzym ausgewählt ist aus L-Galactono-1,4-lacton-Dehydrogenase, L-Galactose-Dehydrogenase, L-Sorboson-Dehydrogenase, L-Gulono-1,4-lacton-Oxidase oder Gemischen davon, und einen Vorläufer oder einen Träger, ausgewählt aus den chemischen oder biologischen Vorläufern von Vitamin C, mit Ausnahme seiner Ester, wobei das Enzym und der Vorläufer so verpackt oder behandelt sind, dass sie nicht miteinander in Kontakt stehen.

2. Zusammensetzung gemäß Anspruch 1, **dadurch gekennzeichnet, dass** der Träger ausgewählt ist aus L-Galactono-1,4-lacton, L-Gulono-1,4-lacton, D-Glucorono-1,4-lacton, D-Glucuronsäure, D-Mannose, D-Galacturonsäure, D-Glucose, D-Galactose, L-Galactose oder Gemischen davon.

3. Zusammensetzung gemäß Anspruch 1 oder 2, **dadurch gekennzeichnet, dass** der Träger L-Galactono-1,4-lacton ist.

4. Zusammensetzung gemäß einem der Ansprüche 1 bis 3, **dadurch gekennzeichnet, dass** das Enzym L-Galactono-1,4-lacton-Dehydrogenase ist.

5. Zusammensetzung gemäß einem der Ansprüche 1 bis 4, **dadurch gekennzeichnet, dass** das Enzym aus einem Extrakt aus Pflanzen, Tieren, Insekten oder Mikroorganismen stammt, insbesondere aus differenzierten oder undifferenzierten Zellen, der in vivo oder in vitro erhalten wird.

6. Zusammensetzung gemäß einem der Ansprüche 1 bis 5, **dadurch gekennzeichnet, dass** das Enzym und der Vorläufer in getrennten Bereichen verpackt werden.

7. Zusammensetzung gemäß einem der Ansprüche 1 bis 6, **dadurch gekennzeichnet, dass** das Enzym und/oder der Vorläufer in verkapselter Form vorliegen.

8. Zusammensetzung gemäß einem der Ansprüche 1 bis 7, **dadurch gekennzeichnet, dass** das Enzym und/oder der Vorläufer in Form von Mikrokapseln oder Mikrogranulaten vorliegen.

9. Zusammensetzung gemäß einem der Ansprüche 1 bis 8, **dadurch gekennzeichnet, dass** das Enzym in Form eines Totalextrakts, einer gereinigten Enzymlösung, als in einer Matrix, insbesondere einer Sol-Gel-Matrix, immobilisiertes Enzym, in fester oder flüssiger Form, in flüssiger oder fester lyophilisierter Form oder in einer Vorrichtung zur kontrollierten Freisetzung vorliegt.

10. Zusammensetzung gemäß einem der Ansprüche 1 bis 9, **dadurch gekennzeichnet, dass** das Enzym in einer Menge von 0,05 bis 30 Gew.-% bezogen auf das Gesamtgewicht der Zusammensetzung vorliegt.

11. Zusammensetzung gemäß einem der Ansprüche 1 bis 10, **dadurch gekennzeichnet, dass** das Enzym in einer Menge von 0,1 bis 10 Gew.-% bezogen auf das Gesamtgewicht der Zusammensetzung vorliegt.

12. Zusammensetzung gemäß einem der Ansprüche 1 bis 11, **dadurch gekennzeichnet, dass** der Vorläufer in einer Menge von 0,01 bis 50 Gew.-% bezogen auf das Gesamtgewicht der Zusammensetzung vorliegt.

13. Zusammensetzung gemäß einem der Ansprüche 1 bis 12, **dadurch gekennzeichnet, dass** der Vorläufer in einer Menge von 0,1 bis 10 Gew.-% bezogen auf der Gesamtgewicht der Zusammensetzung vorliegt.

14. Verwendung von einem oder mehreren Enzymen, ausgewählt aus L-Galactono-1,4-lacton-Dehydrogenase, L-Galactose-Dehydrogenase, L-Sorboson-Dehydrogenase, L-Gulono-1,4-lacton-Oxidase oder Gemischen davon oder eines Extrakts, der diese enthält, zur Herstellung von Vitamin C zur topischen Anwendung, aus einem chemischen oder biologischen Vorläufer von Vitamin C, mit Ausnahme seiner Ester, in einer Zusammensetzung zur topischen Anwendung, wobei das Enzym und der Vorläufer so verpackt oder behandelt sind, dass sie nicht miteinander in Kontakt stehen.

15. Verwendung gemäß Anspruch 14, **dadurch gekennzeichnet, dass** das Enzym L-Galactono-1,4-lacton-Dehydrogenase ist.

16. Verwendung gemäß Anspruch 14 oder 15, **dadurch gekennzeichnet, dass** die Herstellung extemporal stattfindet.

17. Verwendung gemäß einem der Ansprüche 14 bis 16, **dadurch gekennzeichnet, dass** die Herstellung durch in Kontakt bringen des Enzyms oder des Pflanzenextrakts mit einem Träger stattfindet.

18. Verwendung gemäß einem der Ansprüche 14 bis 17, **dadurch gekennzeichnet, dass** das Enzym aus einem Extrakt aus Pflanzen, Tieren, Insekten oder Mikroorganismen stammt, insbesondere aus differenzierten oder undifferenzierten Zellen, der in vivo oder in vitro erhalten wird.

19. Verwendung gemäß einem der Ansprüche 14 bis 18, **dadurch gekennzeichnet, dass** der Träger ausgewählt ist aus L-Galactono-1,4-lacton, L-Gulono-1,4-lacton, D-Glucorono-1,4-lacton, D-Glucuronsäure, D-Mannose, D-Galacturonsäure, D-Glucose, D-Galactose, L-Galactose oder Gemischen davon.

20. Verwendung gemäß einem der Ansprüche 14 bis 19, **dadurch gekennzeichnet, dass** der Träger L-Galactono-1,4-lacton ist.

21. Verfahren zur kosmetischen Behandlung durch Aufbringen einer Zusammensetzung gemäß einem der Ansprüche 1 bis 13 auf die Haut.

22. Verfahren zur kosmetischen Behandlung der Haut, bestehend in dem gleichzeitigen oder zeitverschobenen Aufbringen mindestens eines Enzyms, das in der Lage ist, einen Vorläufer von Vitamin C in Vitamin C umzuwandeln, wobei das Enzym ausgewählt ist aus L-Galactono-1,4-lacton-Dehydrogenase, L-Galactose-Dehydrogenase, L-Sorboson-Dehydrogenase, L-Gulono-1,4-lacton-Oxidase oder Gemischen davon, und mindestens eines Vorläufers von Vitamin C, mit Ausnahme seiner Ester, wobei das Enzym und der Vorläufer so verpackt oder behandelt sind, dass sie nicht miteinander in Kontakt stehen.

23. Verfahren gemäß Anspruch 22, wobei das Enzym L-Galactono-1,4-lacton Dehydrogenase ist.

24. Verfahren gemäß Anspruch 22 oder 23, **dadurch gekennzeichnet, dass** der Vorläufer ausgewählt ist aus L-Galactono-1,4-lacton, L-Gulono-1,4-lacton, D-Glucorono-1,4-lacton, D-Glucuronsäure, D-Mannose, D-Galacturonsäure, D-Glucose, D-Galactose, L-Galactose oder Gemischen davon.

25. Verfahren gemäß einem der Ansprüche 22 bis 24, wobei der Vorläufer L-Galactono-1,4-lacton ist.
